# EUROPÄISCHE PATENTSCHRIFT

(11) **EP 1 305 073 B1**
(45) Veröffentlichungstag und Bekanntmachung des Hinweises auf die Patenterteilung: **12.10.2005**
(21) Anmeldenummer: 01942938.0
(22) Anmeldetag: 29.06.2001
(51) Int. Cl.: A61M 25/00, A61M 5/168

(54) **KATHETERKOPF MIT DURCHFLUSSSENSOR**
CATHETER HEAD HAVING A FLOW SENSOR
TETE DE CATHETER A CAPTEUR DE DEBIT

(30) Priorität: 20.07.2000 DE 10035342
(43) Veröffentlichungstag der Anmeldung: 02.05.2003
(73) Patentinhaber: Disetronic Licensing AG, 3401 Burgdorf (CH)
(72) Erfinder: HEINIGER, Hanspeter, CH-4932 Lotzwil (CH); GEISER, Simone, CH-4900 Langenthal (CH); KRIEFTEWIRTH, Michael, CH-3007 Bern (CH); SCHILTGES, Gilbert, CH-3422 Kirchberg (CH); YEANDEL, Julian, CH-3506 Grosshoechstetten (CH)
(74) Vertreter: Gassenhuber, Andreas
(86) Internationale Anmeldenummer: PCT/CH2001/000405
(87) Internationale Veröffentlichungsnummer: WO 2002/007808

(56) Entgegenhaltungen:
- EP-A- 0 405 148
- WO-A-93/17745
- DE-A- 19 821 723
- US-A- 4 559 831
- US-A- 5 445 622
- US-A- 5 764 539

## Beschreibung

Die vorliegende Erfindung betrifft eine Vorrichtung zum Zuführen von Flüssigkeit in den Körper mit einem Katheterkopf, einer Flüssigkeitszuführung zum Katheterkopfund mit einer Einrichtung zum Messen des Durchflusses der Flüssigkeit.

Um die Verabreichung von Wirkstoffen in den Körper exakt überwachen zu können, ist es notwendig, den Durchfluss der Wirkstoffe durch ein Kathetersystem zu messen. Hierzu sind bereits verschiedene Systeme bekannt. Beispielsweise wird in der US-A 5,764,539 eine Durchflussmessung vorgeschlagen, bei der ein temperaturabhängiger Sensor am Katheterschlauch hinter der Pumpe angebracht wird, dessen Messwerte elektronisch verarbeitet werden.

Ein weiterer solcher Thermo-Strömungssensor, der ebenfalls am flüssigkeitsführenden Schlauch angebracht wird, ist beispielsweise aus der EP 0 405 148 A1 bekannt. Für intravenöse Infusionssysteme schlägt die US-A 5,445,622 ein System zur Strömungsmessung vor, bei dem ein Strömungsindikator, der als separates Handgelenkgerät ausgestaltet ist, an einem Infusionsschlauch angebracht wird. Schließlich ist aus der US-A 5,462,525 ein Durchflusssensor für eine Infusionspumpe bekannt, der an der Pumpe selbst, nämlich am Abflusskanal der Pumpe angeordnet ist.

Der Nachteil aller oben aufgeführten Systeme besteht darin, dass sie Leckagen, die direkt am oder in der Nähe des Injektionspunktes auftreten, in keiner Weise erfassen können. Wenn direkt am Einbringungspunkt in den Körper eine Leckage oder eine Verstopfung auftritt, die dazu führt, dass die Wirkstoffflüssigkeit den Katheter auf eine andere Weise verlässt als vorgesehen, wird ein Durchflusssensor, der gemäß dem Stand der Technik angeordnet ist, diesen Fehler nicht feststellen, und es kann keine entsprechende Gegenmaßnahme (automatisch) eingeleitet werden.

Vor diesem Hintergrund ist es die Aufgabe der vorliegenden Erfindung, eine Vorrichtung zum Zuführen von Flüssigkeit in den Körper bereitzustellen, welche die oben angeführten Nachteile des Standes der Technik überwindet. Insbesondere soll eine zuverlässige Durchflussmessung bereitgestellt werden, die jedweden möglichen Fehler im Wirkstofffluss ermitteln kann und damit die Möglichkeit zu einer sehr genauen und richtigen Durchflusserfassung liefert.

Diese Aufgabe wird durch eine Vorrichtung zum Zuführen von Flüssigkeit in den Körper gelöst, welche die im Anspruch 1, und insbesondere im kennzeichnenden Teil des Anspruchs 1 aufgeführten Merkmale aufweist. Die Unteransprüche zeigen bevorzugte Ausführungsformen der vorliegenden Erfindung auf.

Die Vorteile der vorliegenden Erfindung beruhen auf der Tatsache, dass die Einrichtung zum Messen des Durchflusses ein direkt am bzw. im Katheterkopf angeordneter Durchflusssensor ist. Der Durchflusssensor wird also gemäß der Erfindung an einem Ort des Strömungskanals angeordnet, der unmittelbar an der Schnittstelle zum Körper liegt, also direkt am bzw. im Katheterkopf. Hierdurch wird sichergestellt, dass jedwede Änderung im Durchfluss, die über die gesamte Flüssigkeitszuführung erfolgen könnte, auch vom Durchflusssensor registriert wird; alle Leckagen, Verstopfungen oder die Förderung von Luftblasen sowie der Free-Flow können mittels der erfindungsgemäßen Vorrichtung detektiert werden. Das erfindungsgemäße System arbeitet deshalb sehr viel zuverlässiger als alle bisher bekannten Anordnungen, bei denen Leckagen stromabwärts vom Durchflusssensor nicht registriert werden konnten. Es bietet damit auch unmittelbar die Möglichkeit einer automatischen Regelung in Reaktion auf Durchflussänderungen.

Bei einer Ausführungsform der vorliegenden Erfindung ist der Durchflusssensor stromabwärts von einer Katheterzuleitung des Katheterkopfes und insbesondere zwischen der Katheterzuleitung und einer Verbindungsnadel des Katheterkopfes angeordnet, welche zu einer Katheterkanüle führt. Die Anordnung erfolgt also an dem Punkt, der unmittelbar vor dem Teil des Katheterkopfes liegt, an dem durch genaue Fertigung auszuschließen ist, dass Leckagen auftreten und unmittelbar hinter dem Teil der Zuleitung, in dem Leckagen wegen möglicherweise undichter Adapterverbindungen relativ leicht auftreten können. An der genannten Stelle kann ein Durchflusssensor in miniaturisierter Ausführung angebracht und befestigt werden, und größere Umkonstruktionen des Katheterkopfes sind nicht notwendig.

Um die vom Durchflusssensor erfassten Daten zu überwachen, ist es von Vorteil, wenn an der erfindungsgemäßen Vorrichtung selbst eine Datenübertragungseinrichtung angeordnet wird. Diese Datenübertragungseinrichtung kann unmittelbar mit dem Durchflusssensor integriert sein, und sie verbindet den Durchflusssensor gemäß einer vorteilhaften Ausführungsform der Erfindung mit einer Regelungseinrichtung in einer Pumpe für die Flüssigkeit, mittels der über einen Soll-Istwertvergleich der Durchfluss auf einen vorbestimmten Wert eingeregelt wird. Wie schon oben bemerkt, eignet sich die erfindungsgemäße Vorrichtung aufgrund der optimalen Positionierung des Durchflusssensors und der damit erzielbaren Messrichtigkeit insbesondere für die Einbindung in ein Regelsystem, welches vom Durchflusssensor festgestellte Abweichungen automatisch korrigiert. Liegt beispielsweise momentan ein zu geringer Durchfluss vor oder werden Luftblasen im Kanal festgestellt, so kann über die Regelungseinrichtung ohne weiteres eine größere Förderleistung der Pumpe eingestellt werden, welche dann wieder für eine korrekte Strömungsrate sorgt. Die Verabreichung des Wirkstoffes kann dann wieder planmäßig verlaufen. Besonders bevorzugt wird noch eine Signalausgabeeinrichtung integriert, die bei stärkeren oder nicht korrigierbaren Durchflussabweichungen ein Signal abgibt, das zu einer Überprüfung der Flüssigkeitszuführung auffordert.

In der technischen Ausführung kann der Durchflusssensor einen Analog/Digital-Wandler aufweisen, der auf einem Chip integriert ist und die erfassten Daten digital überträgt.

Die Datenübertragung kann erfindungsgemäß in unterschiedlicher Art und Weise realisiert werden. Bei einer Ausführungsform weist die Datenübertragungseinrichtung Kabel auf, die vom Durchflusssensor im Katheterkopf und in oder an den Bauteilen der Flüssigkeitszuführungen entlang zu einer Verarbeitungseinheit, insbesondere der Regelungseinrichtung an der Pumpe führen, wobei Leitungsübergänge an miteinander zu verbindenden Bauteilen der Flüssigkeitszuführung vorzugsweise durch Kontaktierungen, insbesondere Berührungskontakte realisiert werden.

Die gesamte Datenübertragungseinrichtung ist also hierbei in die Flüssigkeitszuführung integriert, so dass keine zusätzlichen separaten, außen liegenden Verkabelungen benötigt werden. Der Vorteil liegt also insbesondere darin, dass der Patient nur eine einzige Verbindung vom Katheterkopfbis zur Pumpe hat. In technischer Ausführung werden die Kabel direkt in einem Arbeitsgang in den Katheter eingearbeitet und der Katheterschlauch wird zusammen mit den Kabeln gleichzeitig extrudiert. Im Katheterkopf werden die Kabel vom Schlauch getrennt und am Durchflusssensor angelötet oder befestigt. In den Anschlüssen, d.h. in den einzelnen Bauteilen/Adaptern, die mit Kontaktierungen versehen sind, werden die Kabel an den Kontaktierungen befestigt.

Ein weiterer Vorteil dieser Ausführungsform besteht darin, dass durch die Überprüfung der richtigen elektrischen Kontaktierung immer gleichzeitig auch überwacht werden kann, ob die einzelnen, miteinander zu verbindenden Bauteile und Adapter richtig miteinander verbunden wurden.

Gemäß einer weiteren Ausführungsform der vorliegenden Erfindung weist die Datenübertragungseinrichtung Kabel auf, die separat von der Flüssigkeitszuführung zu einer Verarbeitungseinheit, insbesondere der Regelungseinrichtung an der Pumpe führen. Bei dieser Lösung mit separaten Kabeln ist die Flüssigkeitszuführung in einfacher Weise herkömmlich ausgestaltet, und es ist möglich, die Kabel mittels eines Aufsteckadapters an dem Katheterkopf anzuschließen und dort zu befestigen.

Gemäß einer dritten Ausführungsvariante für die Datenübertragungseinrichtung weist diese einen Sender auf, der die vom Durchflusssensor erfassten Daten drahtlos zu einem Empfänger an einer Verarbeitungseinheit, insbesondere der Regelungseinrichtung an der Pumpe, überträgt. Der Sender kann mittels eines Aufsteckadapters an dem Katheterkopf anschließbar und befestigbar sein. Eine solche drahtlose Datenübertragung hat einerseits den Vorteil, dass die Flüssigkeitszushnmgsleitungen nicht mit speziell integrierten Kabeln und Kontaktanschlüssen versehen werden müssen und gewährt andererseits wieder die Möglichkeit, nur eine einzige Leitung zwischen Pumpe und Patient bereitzustellen.

Die Erfindung wird im Weiteren anhand bevorzugter Ausführungsformen mittels der beiliegenden Zeichnungen näher erläutert. Es zeigen:
- Figur 1: einen Katheteranschluss gemäß der vorliegenden Erfindung, wobei die Bauteile der Flüssigkeitszuführung einzeln dargestellt sind;
- Figur 2: einen Katheteranschluss gemäß der vorliegenden Erfindung mit den Bauteilen der Flüssigkeitszuführung im zusammengebauten Zustand;
- Figur 3: die Anordnung nach Figur 2 in einer Seitenansicht; und
- Figur 4: ein Blockdiagramm als Schemadarstellung für den Aufbau einer drahtlosen Datenübertragung zwischen Durchflusssensor und Pumpe.

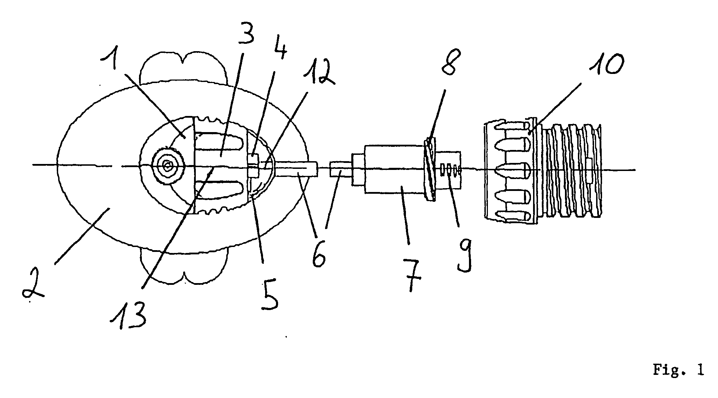

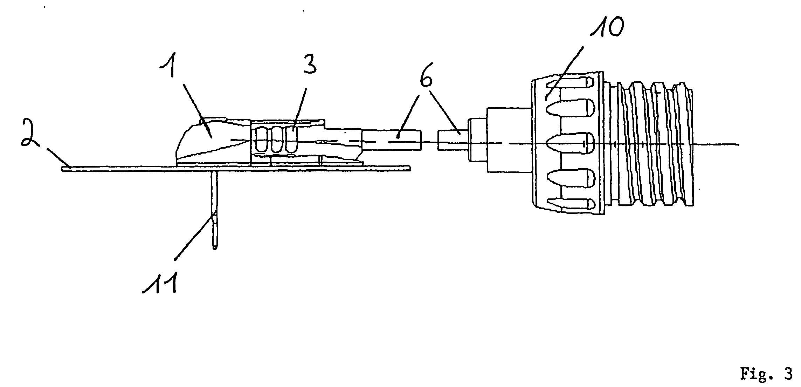

Anhand der Figuren 1 bis 3 sollen im weiteren nunmehr die einzelnen Bauteile eines erfindungsgemäßen Katheteranschlusses beschrieben werden. Der Katheterkopf selbst trägt das Bezugszeichen 1, und er wird mittels eines Befestigungsgewebes (Pflaster) 2 auf der Haut eines Patienten so befestigt, dass die Kanüle 11 (siehe Figur 3) zu dem Ort im Patientenkörper vordringt, zu dem der Wirkstoff gebracht werden soll.

Im Katheterkopf 1 verläuft eine Verbindungsnadel 13, die für die Zuführung des Wirkstoffes in die Kanüle 11 sorgt, und diese Verbindungsnadel 13 liegt unter dem wegnehmbaren Verbindungsstecker 3, auf den ein Adapter (nicht dargestellt) zur Datenübertragung aufgesteckt werden kann.

Zwischen dem Einlass der Verbindungsnadel 13 und der Katheterzuleitung 12 befindet sich der Durchflusssensor 4, ein miniaturisierter Durchflussmesser für kleinste Massenströme. Für den Datenausgang gehen vom Durchflusssensor 4 die Kabel 5 ab, vorzugsweise drei Kabel für die Datenleitung und die Stromversorgung.

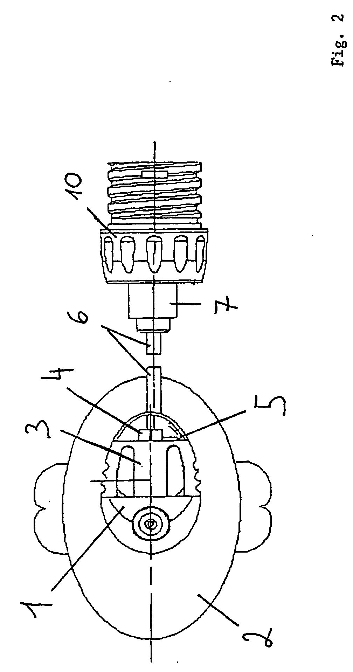

Die dargestellte Ausführungsform ist eine solche, die eine in die Flüssigkeitszuführung integrierte Datenleitung aufweist. Obwohl in den Figuren 1 und 2 nicht speziell dargestellt, laufen die Datenkabel 5 durch den Katheterschlauch 6 hindurch in den Adapter 7 hinein, der mit einem Gewinde 8 und einem rechts davon angeordneten, nicht bezeichneten Luer-Anschluss versehen ist. Am Luer-Anschluss endet die Datenleitung in drei Kontakten 9, die mit nicht sichtbaren Gegenkontakten im Pumpenadapter 10 bei der Verbindung der Bauteile 7 und 10 Kontakt machen. Über weitere integrierte Kabel wird die Datenleitung dann bis zur ebenfalls nicht dargestellten Pumpe weitergeführt.

Die Wirkstoffflüssigkeit strömt somit über die Bauteile 10, 7, 6 und 12 in den Durchflusssensor 4 und dann über die Verbindungsnadel 13 in die Kanüle 11, wo sie im Körper verabreicht wird. Der Durchflusssensor 4 meldet über seine Datenleitung die Strömungsrate zurück, so dass Einregelungen bei Unregelmäßigkeiten im Strömungsfluss vorgenommen werden können. Bei der dargestellten Ausführungsform ist wegen der Integrierung der Datenleitung in die Flüssigkeitsleitung keine separate und eventuell störende Datenleitung notwendig.

Die Figur 2 zeigt die Anordnung gemäß Figur 1 in einem Zustand, in dem die Adapter 7 und 10 schon zusammengebaut sind und in der Figur 3 ist dieser Zustand in der Seitenansicht erkennbar.

An dieser Stelle soll nochmals auf den Verbindungsstecker 3 Bezug genommen werden, wie er in den Figuren 1 bis 3 dargestellt ist. Es besteht grundsätzlich die Möglichkeit, die Kabel 5 nicht durch die Zuleitung zu führen, sondern an den Verbindungsstecker 3 anzuschließen und über diese Verbindung ein separates Datenleitungskabel zur Datenübertragung wegzuführen. In diesem Fall ist es nicht notwendig, die Flüssigkeitszuleitung relativ aufwendig mit integrierten Kabeln und Anschlüssen herzustellen, sondern es kann eine herkömmliche Flüssigkeitszuleitung verwendet werden.

Der Durchflusssensor ist gemäß einem sogenannten Packaging-Konzept konstruiert. Verstärker und Analog/Digital-Wandler werden auf dem Chip integriert, so dass außer dem Chip, der den eigentlichen Sensor enthält, keine weiteren elektronischen Bauteile notwendig sind. Die Übertragung der Daten vom Sensor erfolgt digital.

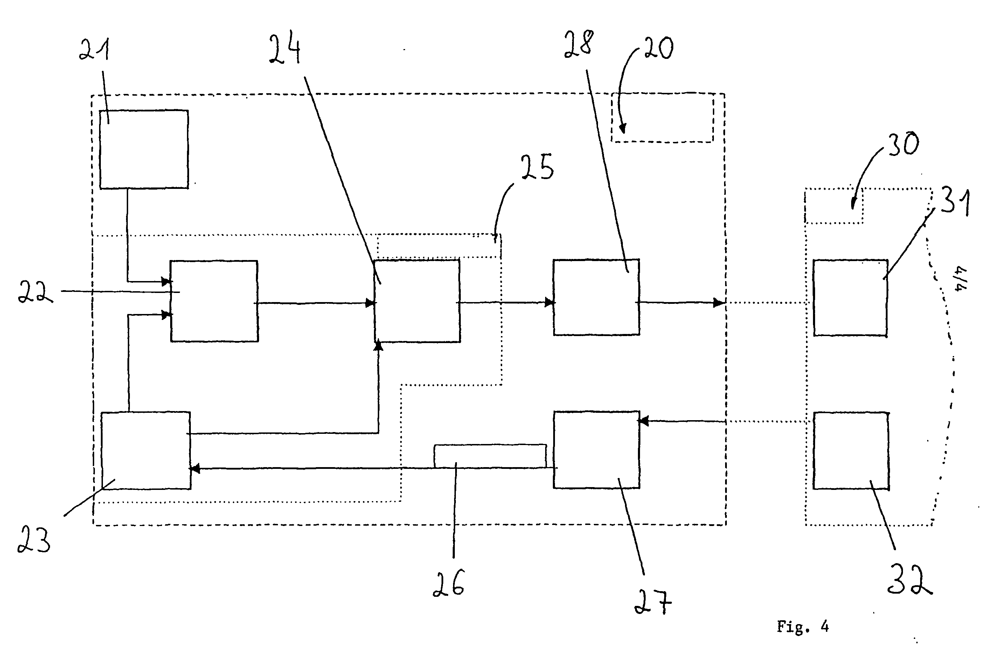

An den in den Figuren 1 bis 3 dargestellten Verbindungsstecker 3 kann gemäß einer weiteren Ausführungsform der Erfindung auch ein Sender zur drahtlosen Datenübertragung angebracht werden, der dann über die Kabel 5 die Daten des Durchflusssensors 4 erhält und drahtlos weitergibt, also beispielsweise zur Pumpe sendet. Der Sender ist mit einer entsprechenden Elektronik (siehe Figur 4) ausgestattet, die es erlaubt, die Daten verschlüsselt nur der jeweils zugeordneten Pumpe zu übersenden. Der Sender kann mit einer auswechselbaren Batterie als Energiequelle ausgestattet sein und kann auf den Katheterkopf montiert und wieder demontiert werden.

Die schon angesprochene Figur 4 zeigt ein Blockdiagramm für die Senderelektronik (Sender) 20 und den Pumpenein- und ausgang 30. Die Senderelektronik 20 ist wie vorher beschrieben mit dem Durchflusssensor 5 integriert. Der in Figur 4 rechts dargestellte Pumpenteil 30 weist einen Digitaltransmitter 32 und einen Digitalempfänger 31 auf, jeweils für die drahtlos übertragenen Digitalsignale des Senders 20. Im Sender 20 ist der Sensor-Operationsverstärker 21 dargestellt, der die vom Sensor 4 erhaltenen Signale verstärkt und an den A/D-Wandler 22 weitergibt. Der A/D-Wandler ist Bestandteil eines Mikrocontrollers 25, der noch den Timingssignalgenerator 23 und den Datenkodierer 24 umfasst. Aus dem A/D-Wandler werden die Daten des Sensors 4, verstärkt im Operationsverstärker 21, in den Digitaltransmitter 28 des Senders 20 übergeben, der sie dann weitersendet zum Digitalempfänger 31 der Pumpe 30.

In einer nicht dargestellten Elektronik der Pumpe 30 werden die Durchflussdaten mittels eines Ist-Sollwertvergleiches überarbeitet und, wenn ein Korrektursignal notwendig ist, wird dieses über den Digitaltransmitter 32 zum Digitalempfänger 27 des Senders 20 übergeben. Diese Korrekturdaten 26 werden dann an den Timing-Signalgenerator 23 weitergegeben, der über Ausgänge an den A/D-Wandler und an den Kodierer 24 - auf direktem und/oder indirektem Weg - den neuen Ist-Zustand über den Digitaltransmitter 28 an die Pumpe sendet. Wenn der neue Ist-Zustand unter Inbetrachtziehung der durchgeführten Korrektur, also einer Änderung der Pumpen-Flüssigkeitsabgabe, im vorgeschriebenen Bereich liegt, erfolgt die Medikamentenverabreichung wieder plangemäß. Wenn dies nicht der Fall ist, kann ein Signal ausgegeben werden, welches zur manuellen Überprüfung des gesamten Kathetersystems auffordert.

Zu dem Diagramm der Figur 4 ist noch folgendes anzumerken: Die lokale Energieversorgung für den Durchflusssensor 4/Sender 20 (um den Sensor zu betreiben, ist die Erzeugung einer höheren Spannung notwendig) ist als Teil des Sensor-Operationsverstärkermoduls 21 integriert. Deshalb kann die Batteriespannung (im Bereich von 1,2 bis 1,5 V) als einzige globale Energieversorgung verwendet werden. Der Operationsverstärker 21 gibt Signale ab, die in dem normalen Bereich von-10 bis +10 V liegen. Der Timing-Signalgenerator 23 kann geeignete Signale erzeugen, um ein Sampling der analogen Signale durch den A/D-Wandler 22 zu steuern. Ferner kann der Timing-Signalgenerator 23 geeignete Signale erzeugen, um die Kombination der gesampelten Vorrichtungsidentifikations-/Strömungsdaten vor der digitalen Transmission zu regeln. Es existiert eine geeignete serielle Schnittstelle zwischen dem Mikrocontroller 25 und dem digitalen Empfänger 31 sowie dem digitalen Transmitter 32, um eine Datenverarbeitung zu gestatten. Ferner existiert eine billige Kodierung. Diese würde beispielsweise bestehen aus einer Kombination von Metallkontakten, die auf einer Leiterplatte aufgedruckt sind, und einer leitenden Auftragsfarbe. Dieser Kode wird ein Teil der übertragenen Daten, die durch den Mikrocontroller 25 erzeugt werden.

Um den Mikrocontroller zu betreiben, kann ein geeignetes Programm entwickelt werden, das alle notwendigen Koordinationen im Datenaufbau, bei der Kommunikation und im Stromversorgungs-Management übemimmt.

Die Erfindung gestattet in allen Ausführungsformen mit der unmittelbaren Anordnung des Durchflusssensors am Katheterkopf eine hochgradig messrichtige und fehlerfreie Strömungsratenmessung, insbesondere was Leckagen und/oder Verstopfungen in fehleranfälligen Bauteilen, wie Anschlussadaptern in der Flüssigkeitszuführung vor dem Katheterkopf betrifft.

### Bezugszeichenliste

- 1: Katheterkopf
- 2: Befestigungsgewebe (Pflaster)
- 3: Verbindungsstecker
Sender am Verbindungsstecker 3 (nicht gezeigt)
- 4: Durchflusssensor
- 5: Kabel/Datenkabel
- 6: Katheterschlauch
- 7: Adapter
- 8: Gewinde am Adapter
Luer-Anschluss am Adapter (nicht gezeigt)
- 9: Kontakte am Luer-Anschluss
- 10: Pumpenadapter
Gegenkontakte im Pumpenadapter
- 11: Kanüle
- 12: Katheterzuleitung
- 13: Verbindungsnadel 13 im Katheterkopf
- 20: Sender/Senderelektronik
- 21: Sensor-Operationsverstärker
- 22: A/D Wandler
- 23: Timingssignalgenerator
- 24: Datenkodierer
- 25: Mikrocontroller
- 28: Digitaltransmitter des Senders
- 30: Pumpenein- und Ausgang
- 31: Digitalempfänger

## Patentansprüche

1. Vorrichtung zum Zuführen von Flüssigkeit in den Körper mit einem Katheterkopf (1), einer Flüssigkeitszuführung (6, 7, 10) zum Katheterkopf (1) und mit einer Einrichtung zum Messen des Durchflusses der Flüssigkeit, **dadurch gekennzeichnet, dass** die Einrichtung zum Messen des Durchflusses ein direkt am bzw. im Katheterkopf (1) angeordneter Durchflusssensor (4) ist.

2. Vorrichtung nach Anspruch 1, **dadurch gekennzeichnet, dass** der Durchflusssensor (4) stromabwärts von einer Katheterzuleitung (12) des Katheterkopfes (1) und insbesondere zwischen der Katheterzuleitung (12) und einer Verbindungsnadel (13) des Katheterkopfs angeordnet ist, welche zu einer Katheterkanüle (11) führt.

3. Vorrichtung nach Anspruch 1 oder 2, **dadurch gekennzeichnet, dass** sie eine Datenübertragungseinrichtung (5, 9) für die vom Durchflusssensor (4) erfassten Daten aufweist.

4. Vorrichtung nach Anspruch 3, **dadurch gekennzeichnet, dass** die Datenübertragungseinrichtung (5, 9) den Durchflusssensor (4) mit einer Regelungseinrichtung in einer Pumpe für die Flüssigkeit verbindet, mittels der über einen Soll-Istwertvergleich der Durchfluss auf einen vorbestimmten Wert eingeregelt wird.

5. Vorrichtung nach Anspruch 3 oder 4, **dadurch gekennzeichnet, dass** der Durchflusssensor (4) einen Analog/Digitalwandler aufweist, der auf einem Chip integriert ist, und die erfassten Daten digital überträgt.

6. Vorrichtung nach einem der Ansprüche 3 bis 5, **dadurch gekennzeichnet, dass** die Datenübertragungseinrichtung Kabel (5) aufweist, die vom Durchflusssensor (4) im Katheterkopf (1) und in oder an den Bauteilen (7, 10) der Flüssigkeitszuführung entlang zu einer Verarbeitungseinheit, insbesondere der Regelungseinrichtung an der Pumpe, führen, wobei Leitungsübergänge an miteinander zu verbindenden Bauteilen der Flüssigkeitszuführung vorzugsweise durch Kontaktierungen, insbesondere Berührungskontakte (9) realisiert werden.

7. Vorrichtung nach einem der Ansprüche 3 bis 5, **dadurch gekennzeichnet, dass** die Datenübertragungseinrichtung Kabel aufweist, die separat von der Flüssigkeitszuführung zu einer Verarbeitungseinheit, insbesondere der Regelungseinrichtung an der Pumpe führen.

8. Vorrichtung nach Anspruch 7, **dadurch gekennzeichnet, dass** die Kabel mittels eines Aufsteckadapters an dem Katheterkopf (1) anschließbar und befestigbar sind.

9. Vorrichtung nach einem der Ansprüche 3 bis 5, **dadurch gekennzeichnet, dass** die Datenübertragungseinrichtung einen Sender aufweist, der die vom Durchflusssensor (4) erfassten Daten drahtlos zu einem Empfänger an einer Verarbeitungseinheit, insbesondere der Regelungseinrichtung an der Pumpe, überträgt.

10. Vorrichtung nach Anspruch 9, **dadurch gekennzeichnet, dass** der Sender mittels eines Aufsteckadapters an dem Katheterkopf (1) anschließbar und befestigbar ist

## Claims

1. A device for supplying liquid into the body, comprising a catheter head (1), a liquid supply (6, 7, 10) to said catheter head (1) and a means for measuring the flow of the liquid, **characterised in that** said means for measuring the flow is a flow sensor (4) arranged directly on or in the catheter head (1).

2. The device as set forth in claim 1, **characterised in that** said flow sensor (4) is arranged downstream of a catheter supply line (12) of the catheter head (1) and in particular between the catheter supply line (12) and a connecting needle (13) of the catheter head which leads to a catheter cannula (11).

3. The device as set forth in claim 1 or 2, **characterised in that** it comprises a data transfer means (5, 9) for the data captured by the flow sensor (4).

4. The device as set forth in claim 3, **characterised in that** said data transfer means (5, 9) connects the flow sensor (4) to a regulating means in a pump for the liquid, by means of which the flow is regulated to a predetermined value by comparing desired and actual values.

5. The device as set forth in claim 3 or 4, **characterised in that** the flow sensor (4) comprises an analogue/digital converter which is integrated on a chip and digitally transfers the data captured.

6. The device as set forth in any one of claims 3 to 5, **characterised in that** the data transfer means comprises cables (5) which lead from the flow sensor (4) in the catheter head (1) and in or on the components (7, 10) along the liquid supply to a processing unit, in particular the regulating means on the pump, wherein line junctions at components of the liquid supply to be connected to each other are preferably realised by contacts, in particular touching contacts (9).

7. The device as set forth in any one of claims 3 to 5, **characterised in that** the data transfer means comprises cables which lead separately from the liquid supply to a processing unit, in particular the regulating means on the pump.

8. The device as set forth in claim 7, **characterised in that** it is possible to connect the cables to the catheter head (1) and fix them there by means of a plug-on adaptor.

9. The device as set forth in any one of claims 3 to 5, **characterised in that** the data transfer means comprises a transmitter which wirelessly transfers the data captured by the flow sensor (4) to a receiver on a processing unit, in particular the regulating means on the pump.

10. The device as set forth in claim 9, **characterised in that** it is possible to connect the transmitter to the catheter head (1) and fix it there by means of a plug-on adaptor.

## Revendications

1. Dispositif pour faire pénétrer un liquide dans un corps avec une tête à cathéter (1), une conduite d'alimentation de liquide (6, 7, 10) vers la tête à cathéter (1) et avec un dispositif pour mesurer le débit du liquide, **caractérisé en ce que** le dispositif pour mesurer le débit est un capteur de débit (4) disposé directement sur ou dans la tête à cathéter (1).

2. Dispositif selon la revendication 1, **caractérisé en ce que** le capteur de débit (4) est disposé en aval d'une conduite de cathéter (12) de la tête à cathéter (1) et en particulier entre la conduite de cathéter (12) et une aiguille de liaison (13) de la tête à cathéter, laquelle conduit à une canule à cathéter (11).

3. Dispositif selon la revendication 1 ou 2, **caractérisé en ce qu'**il comporte un dispositif de transmission de données (5, 9) pour les données saisies par le capteur de débit (4).

4. Dispositif selon la revendication 3, **caractérisé en ce que** le dispositif de transmission de données (5, 9) relie le capteur de débit (4) à un dispositif de régulation situé dans une pompe pour le liquide, au moyen duquel le débit est réglé sur une valeur prédéfinie par une comparaison valeur réelle-valeur prescrite.

5. Dispositif selon la revendication 3 ou 4, **caractérisé en ce que** le capteur de débit (4) comporte un convertisseur analogique/numérique qui est intégré sur une puce et qui transmet les données de manière numérique.

6. Dispositif selon l'une des revendications 3 à 5, **caractérisé en ce que** le dispositif de transmission de données comporte des câbles (5), qui conduisent du capteur de débit (4) situé dans la tête à cathéter (1) et dans ou le long des pièces de construction (7, 10) de la conduite d'alimentation du liquide vers une unité de traitement, en particulier au dispositif de régulation situé sur la pompe, des transitions de conduite sur les pièces de construction à relier entre elles de la conduite d'alimentation du liquide étant réalisées de préférence par des métallisations, en particulier des contacts d'appuis (9).

7. Dispositif selon l'une des revendications 3 à 5, **caractérisé en ce que** le dispositif de transmission de données comporte des câbles qui conduisent, séparément de la conduite d'alimentation du liquide, à une unité de traitement, en particulier au dispositif de régulation situé sur la pompe.

8. Dispositif selon la revendication 7, **caractérisé en ce que** les câbles peuvent être raccordés et fixés au moyen d'un adaptateur à fiche à la tête à cathéter (1).

9. Dispositif selon l'une des revendications 3 à 5, **caractérisé en ce que** le dispositif de transmission de données comporte un émetteur, qui transmet sans fil les données saisies par le capteur de débit (4) à un récepteur situé sur une unité de traitement, en particulier au dispositif de régulation situé sur la pompe.

10. Dispositif selon la revendication 9, **caractérisé en ce que** l'émetteur peut être raccordé et fixé au moyen d'un adaptateur à fiche à la tête de cathéter (1).
